# EUROPEAN PATENT APPLICATION

(11) **EP 2 796 470 A2**
(43) Date of publication of application: **29.10.2014**
(21) Application number: 14162534.3
(22) Date of filing: 31.03.2014
(51) Int. Cl.: C07K 16/44, G01N 33/536, G01N 33/543

(54) **Anti-canine N-terminal pro-atrial natriuretic peptide antibody, and immunological measurement method and immunologically measuring kit using the same**

(30) Priority: 05.04.2013 JP 2013079431; 07.08.2013 JP 2013163755
(71) Applicant: Shibayagi Co., Ltd., Shibukawa-shi, Gumma-ken 377-0007 (JP)
(72) Inventor: Kojima, Masaaki, Gumma-ken, 377-0007 (JP); Hachisu, Tatsuyuki, Gumma-ken, 377-0007 (JP)
(74) Representative: Gille Hrabal

(57) **Abstract**

The anti-canine N-terminal pro-atrial natriuretic peptide antibodies (anti-canine NT-proANP antibodies) according to the present invention comprise anti-canine NT-proANP recognizing a partial portion or a whole portion of the amino acid sequence 31 to 67 of canine NT-proANP and anti-canine NT-proANP recognizing a partial portion or a whole portion of the amino acid sequence 68 to 98 thereof. The anti-canine NT-proANP antibodies are useful for immunologically measuring canine NT-proANP involved in heart diseases and infections of companion animals such as dogs, such as heart failure, mitral valve insufficiency and filariasis. The immunochromatographic assay using the immunochromatographic means such as immunochromatographic strip is extremely convenient and simple for measuring canine NT-proANP and is used as hand-carried and simple devices.

## Description

### TECHNICAL FIELD

The present invention relates to an anti-canine N-terminal pro-atrial natriuretic peptide antibody, and an immunological measurement method and an immunologically measuring kit using the same. More particularly, the present invention relates to an anti-canine N-terminal pro-atrial natriuretic peptide antibody recognizing a canine N-terminal pro-atrial natriuretic peptide (hereinafter referred to also as "canine NT pro-ANP") different from human NT pro-ANP, an immunological measurement method using the same for the measurement of the canine proANP, a method for the detection of heart diseases and filariasis of companion animals such as dogs and cats, and an immunologically measuring kit for measuring the canine pro-ANP.

### BACKGROUND TECHNOLOGY

Recently, eating habits and living environments of companion animals such as dogs and cats have changed, like humans, resulting in a dramatic acceleration of aging and a rapid increase in lifestyle-related diseases such as heart diseases. It is therefore needed to detect symptoms of heart diseases in companion animals as early as possible and provide treatment appropriate for the symptoms of the heart diseases of the companion animals such as dogs and cats, just like heart diseases in human patients.

In order to treat heart diseases of companion animals in accordance with their degrees, kinds, etc., the symptoms of the heart diseases have to be checked and diagnosed in detail. For the early examination of the heart diseases in companion animals, it is preferred embodiment, as a matter of course, to perform medical examination using test reagents and examination protocols exclusive for use in animals. The fact is, however, that the reagents and protocols which have been developed and used for physiological examination of human diseases are used as they are for physiological examination for animal diseases. Therefore, a development of protocols for examination and diagnosis appropriate and exclusive for companion animals has been demanded.

It has been reported that atrial natriuretic peptide (ANP) is expected to be clinically applicable to various human diseases such as hypertension, edematous disorders, renal insufficiency, cardiac insufficiency, etc. owing to its potent diuretic and natriuretic activities or vasodilation action, and it showed an improved effect on cardiac function disorders for patients with congestive heart failure (Non-Patent Literature Document 1). It is also reported that the ANP has already been clinically applied widely as a first option drug for acute cardiac insufficiency (Guidelines for Treatment of Acute Cardiac Insufficiency, 2006, Revised Edition). Moreover, recently, the ANP has been reported about its clinical studies on acute renal failure and myocardial infarction (reperfusative disorders) (Non-Patent Literature Documents 2 and 3).

The pro-atrial natriuretic peptide (proANP) consisting of 126 amino acids is biosynthesized by the atrial cardiac muscle cells and accumulated mainly in intraatrial granules. The ANP is caused to be decomposed in response to the stimulation by atrial pressure into proANP fragments represented by the amino acid sequence 1 to 98 (NT-proANP) and by the amino acid sequence 99 to 126 (ANP), resulting in secretion into the blood. The ANP concentration in the blood has already been applied to diagnosis by an immunological measurement method as a marker for cardiac insufficiency and chronic retinal failure. The NT-proANP has been reported, on the one hand, for example, to be a useful marker for septicemia, while it has been reported, on the other hand, to become a risk factor for cardiac insufficiency (Non-Patent Literature Document 5). There is a report, too, that an increased amount of the ANP was recognized in dogs with congestive heart failure (Non-Patent Literature Document 6).

It is also reported that the NT-proANP (1-98) is further decomposed into three fragments, i.e., NT-proANP (1-30), NT-proANP (31-67) and NT-proANP (79-98), in the blood circulation (see, e.g., Non-Patent Literature Document 7). These pro-ANP fragments are indicated each to be increased in the blood in various diseases so that they may work as significant markers for those diseases (see, e.g., Non-Patent Literature Document 7).

The method for measuring proANP is per se known, which is directed to a method for immunologically measuring γ-proANP using a monoclonal antibody recognizing a portion 1 to 25 of γ-ANP (proANP) (Patent Document 1).

It has also been reported regarding a sandwich-type assay for measuring human NT-proANP using two kinds of monoclonal antibodies recognizing different portions of human NT-proANP (Non-Patent Literature Document 8 and Patent Documents 2, 3).

Non-Patent Literature Document 8 discloses the sandwich assay for immunologically measuring human NT-proANP using a monoclonal antibody recognizing the amino acid sequence 1 to 30 of human NT-proANP and a monoclonal antibody recognizing the amino acid sequence 79 to 98 thereof.

Patent Document 2 discloses the method for measuring human NT-proANP, which comprises an incubation step for incubating a sample, a first antibody recognizing a particular site of NT-proANP, and a second antibody recognizing a NT-proANP site different from the particular site thereof, and a detection step for detecting the resulting antigen-antibody complex. The first and second antibodies used in this document recognize a portion of the NT-proANP containing the amino acid sequence 43 to 66 thereof, respectively.

Patent Document 3 discloses an immunological measurement method for measuring human NT-proANP using a first monoclonal antibody recognizing an amino acid sequence 1 to 30 of NT-proANP and a second monoclonal antibody recognizing amino acid sequence 65 to 84 of NT-proANP.

Patent Document 4 discloses an improved sandwich immunoassay for identifying a partial NT-proANP sequence in diagnosing heart diseases and septicemia, which uses two antibodies connecting specifically to partial sequence of an intermediate region of NT-proANP (a region extending from amino acid sequence 53 to 83 of the NT-proANP).

As disclosed in Non-Patent Literature Document 9, atrial natriuretic peptide (ANP) may be useful for diagnosing cardiac insufficiency of animals such as dogs and further become a potent tool for predicting heart failure of dogs. This reference reports to the extent that canine NT-proANP has been assessed as useful as a preclinical marker for heart diseases of dogs in a certain study, while an objection was raised to that assessment. Even this study was carried out using a commercially available kit containing an antibody to amino acid sequence 1 to 98 of human NT-proANP, but not using an antibody specific to canine NT-proANP.

Non-Patent Literature Document 10 has further reported the result of measurement by human NT-proANP for heart failure of dogs using a RIA kit containing an anti-serum antibody to amino acid residues 80 to 96 of human NT-proANP. Further, Non-Patent Literature Document 11 discloses, similarly, the result of measurement by RIA for canine mitral valve insufficiency using amino acid residues 79 to 98 of human NT-proANP.

It should be noted herein that all these results disclosed in the prior references are obtained each by a measurement system using an antibody to human NT-proANP (1 to 98), but by a measurement system using no antibody specific to canine NT-proANP. Therefore, the test results disclosed by the above references cannot be said to reflect accurate measurement results to be achieved for companion animals such as dogs. A development of a measurement system, accordingly, has been demanded, which uses an antibody specific to canine NT-proANP of companion animals such as dogs and reflects the results to be expected to be achieved by using a canine NT-proANP-specific antibody.

### PRIOR ART REFERENCES

### Patent Documents:

Patent Document 1: Japanese Patent No. 2,561,513.
Patent Document 2: Japanese Patent Application Publication No. 1997-226,919.
Patent Document 3: Japanese Patent Application Publication No. 2005-114,480.
Patent Document 4: Japanese Patent Application Publication No. 2006-523,298.

### Non-Patent Literature Documents:

Non-Patent Literature Document 1: Saito, Y., et al., Circulation 1987; 76: 1362-74.
Non-Patent Literature Document 2: Kitakaze, M., et al., Lancet 2007; 370: 1483-93.
Non-Patent Literature Document 3: Kasamino acid sequence. et al., Eur. Heart J. 2008; 29; 1485-94.
Non-Patent Literature Document 4: Hoffmann, U., et al. Clin. Lab. 2005;51 (7-8):373-9.
Non-Patent Literature Document 5: Berger R, et al., European Journal of Clinical Investigation, 2005, 35 (1), 24-31.
Non-Patent Literature Document 6: Asano, K., et al., Journal of Veterinary Medical Science, 1999, 61,523-529.
Non-Patent Literature Document 7: Daggubati et al., Cardiovascular Research (1997), 246-255.
Non-Patent Literature Document 8: Upsala Journal of Medical Science 102, 99-108 (1997).
Non-Patent Literature Document 9: Tarnow, I., et al.: The Veterinary Journal 180 (2009) 195-201.
Non-Patent Literature Document 10: Prosek, R., et al. J. Vet. Intern. Med. 2007; 21: 238-242.
Non-Patent Literature Document 11: Haggstrom, J., et al. Journal of Veterinary Cardiology, Vol. 2, No. 1, May 2000, pp. 7-16.

### SUMMARY OF THE INVENTION

In order to meet such demands, as a result of extensive review and studies, the present inventors have succeeded in preparing an antibody specific to canine NT-proANP, capable of recognizing a portion of the amino acid sequence of the canine NT-proANP different from that of human NT-proANP, as well as an immunological measurement method for immunologically measuring the canine NT-proANP using the antibody.

Therefore, the main object of the present invention is to provide an anti-canine NT-proANP antibody, which comprises an antibody recognizing a portion of the amino acid sequence of the canine NT-proANP different from that of human NT-proANP, preferably a monoclonal antibody.

In a preferred embodiment, the present invention provides at least two antibodies recognizing a portion of the amino acid of the canine NT-proANP different from that of the human NT-proANP, which comprises an antibody recognizing a partial portion or a whole portion of the amino acid sequence 31 to 67 of the canine NT-proANP and a partial portion or a whole portion of the amino acid sequence 68 to 98 thereof, respectively.

In another preferred embodiment of the present invention, there is provided hybridoma strain producing the antibody to canine NT-proANP as described above.

The present invention has another object to provide an immunological measurement method for immunologically measuring canine NT-proANP of a companion animal, i.e., non-human mammal, such as dog or cat, using the canine NT-proANP antibody.

In a preferred embodiment of the present invention, there is provided the immunological measurement method for immunologically measuring the canine NT-proANP using plural antibodies, preferably two antibodies, each recognizing a portion of the amino acid sequence of the canine NT-proANP different from that of the human NT-proANP. They may include, but not be limited to, an antibody capable of recognizing a partial portion or a whole portion of the amino acid sequence 31 to 67 of the canine NT-proANP and an antibody capable of recognizing a partial or a whole portion of the amino acid sequence 68 to 98 thereof.

In a preferred embodiment, the present invention has another object to provide the immunological measurement method selected from radioimmunoassay, enzyme immunoassay, coagulation assay, luminescence immunoassay, etc., preferably enzyme immunoassay such as ELISA (enzyme-linked immunosorbent assay) or immunochromatography.

A further object of the present invention is to provide an immunologically measuring kit for immunologically measuring canine NT-proANP as an antigen in a sample as an object of measurement by the immunological measurement method of the present invention. In a preferred embodiment of the present invention, the immunologically measuring kit may comprise a support means such as a plate on which the antigen is immobilized, a first antibody being conjugated with the antigen and selected from an anti-canine NT-proANP (31-67) antibody raised to the amino acid sequence 31 to 67 of the canine NT-proANP, preferably an anti-canine NT-proANP (32-40) antibody to the amino acid sequence 32 to 40 thereof, and an anti-canine NT-proANP (68-98) antibody raised to the amino acid sequence 68 to 98 thereof, preferably an anti-canine NT-proANP (74-82) antibody to the amino acid sequence 74 to 82 thereof, a second antibody different from the first antibody, selected from the anti-canine NT-proANP (31-67) antibody, preferably the anti-canine NT-proANP (32-40) antibody, and the anti-canine NT-proANP (68-98) antibody to the amino acid sequence 68 to 98 thereof, preferably the anti-canine NT-proANP (74-82) antibody, and labeled with an enzyme or an enzyme labeled with a biotin-conjugated avidin, the enzyme including, for example, hydrogen peroxidase or horseradish peroxidase and the avidin of the biotin-conjugated avidin including, for example, avidin and streptoavidin, and a chromogenic substrate.

The present invention has a still further object to provide a method of detecting a disease including, but being not limited to, heart diseases such as mitral valve insufficiency, cardiac insufficiency, etc. of non-human mammals such as dogs by measuring the canine NT-proANP with the canine NT-proANP antibody in accordance with the immunological measurement method of the present invention.

The present invention has a still further object to provide a method for detecting an infection infesting the heart of a companion animal, such as infectious diseases, preferably filariasis, using the canine NT-proANP antibody in accordance with the immunological measurement method of the present invention.

The present invention has a still further object to provide an immunological measuring kit, comprising an immunochromatographic means for immunochromatographically measuring canine NT-proANP of a non-human mammal such as a dog as an antigen as the object of measurement, the immunochromatographic means comprising an immunochromatographic strip made of a piece of porous material or sintered polymer, a first antibody and a second antibody different from the first antibody, each selected from an anti-canine NT-proANP (31-67) antibody to the amino acid sequence 31 to 67 of the canine NT-proANP, preferably an anti-canine NT-proANP (32-40) antibody to the amino acid sequence 32 to 40 thereof, and an anti-canine NT-proANP (68-98) antibody to the amino acid sequence 68 to 98 thereof, preferably an anti-canine NT-proANP (74-82) antibody to the amino acid sequence 74 to 82 thereof, and a colored substance such as colloidal metal particles such as colloidal gold particles, fluorescent or magnetic labeled particles, preferably colloidal metal particles, most preferably colloidal gold particles, wherein the antigen in the sample is conjugated with the first antibody labeled with the colored substance and the antigen conjugated therewith is further conjugated with the second antibody, accumulating in the test line changing color and consequently resulting in visualizing the color change on the test line and determining the presence of the antigen of question in the sample.

In a preferred embodiment of the present invention, the immunochromatographic strip is provided with a sample pad, a conjugate pad, and a membrane with a test line, wherein the sample is poured onto the sample pad, the conjugate pad is held with the first antibody labeled with the colored substance so as to enable conjugation of the antigen in the sample with the first antibody labeled with the colored substance to form an antigen-first antibody conjugate on migration of the sample from the sample pad to the conjugate pad, the antigen-first antibody conjugate formed in the conjugate pad is further conjugated with the second antibody immobilized on the test line of the migration membrane on migration from the conjugate pad through the migration membrane to the test line thereof, changing color of the test line and visualizing the test line to confirm the presence of the antigen in the sample.

In a still further aspect of the present invention, there is provided an immunochromatographic assay comprising:
placing a first antibody labeled with a colored substance on the conjugate pad of the immunochromatographic pad, selected from the anti-canine NT-proANP (31-67) antibody, preferably the anti-canine NT-proANP (32-40) antibody, and the anti-canine NT-proANP (68-98) antibody, preferably the anti-canine NT-proANP (74-82) antibody, the colored substance being selected from colored metal particles, fluorescent or magnetic labeled particles,
immobilizing a second antibody different from the first antibody, selected from the anti-canine NT-proANP (31-67) antibody, preferably the anti-canine NT-proANP (32-40) antibody, the second antibody the anti-canine NT-proANP (68-98) antibody, preferably the anti-canine NT-proANP (74-82) antibody, on the test line of the immunochromatographic strip, provided however that when the first antibody is the anti-canine NT-proANP (31-67) antibody, preferably the anti-canine NT-proANP (32-40) antibody, the second antibody is the anti-canine NT-proANP (68-98) antibody, preferably the anti-canine NT-proANP (74-82) antibody, and vice versa,
bringing a sample containing canine NT-proANP as the antigen of a non-human mammal such as a dog into contact with the sample pad,
conjugating the antigen contained in the sample migrated from the sample pad to the conjugate pad of the migration membrane containing the colored substance-labeled first antibody, resulting in the formation of an antigen-first antibody conjugate by antigen-antibody reaction,
conjugating the conjugate of the colored substance-labeled first antibody with the second antibody immobilized on the test line of the migration membrane of the immunochromatographic strip, resulting in the formation of a first antigen-antigen-second antibody conjugate on the test line of the migration membrane, and
visualizing the colored substance of the colored substance-labeled first antibody of the first antigen-antigen-second antibody conjugate accumulated in the test line of the migration membrane, resulting in changing color of the test line and confirming the presence of the canine NT-proANP in the sample.

In a still further preferred embodiment of the present invention, the colored substance is colored metal particles, preferably colored metal colloidal particles, more preferably colloidal gold particles.

In order to achieve the objects as described above, the present invention in a major aspect provides the anti-canine NT-proANP antibody comprising the antibody recognizing a portion of the amino acid sequence of canine NT-proANP different from that of human NT-proANP.

The present invention in a preferred embodiment provides the anti-canine NT-proANP antibody comprising an anti-canine NT-proANP (31-67) antibody realizing a partial portion or a whole portion of the amino acid sequence 31 to 67 and/or an anti-canine NT-proANP (68-98) antibody recognizing a partial portion or a whole portion of the amino acid sequence 68 to 98 of the canine NT-proANP.

In a further preferred embodiment of the present invention, there is provided the anti-canine NT-proANP (31-67) antibody which specifically recognizes the amino acid sequence 32 to 40 of the canine NT-proANP and the anti-canine NT-proANP (68-98) antibody which specifically recognizes the amino acid sequence 74 to 82 thereof.

In a further preferred embodiment of the present invention, the anti-canine NT-proANP (31-67) antibody may be a polyclonal antibody or a monoclonal antibody, each recognizing the amino acid sequence 32 to 40 of the canine NT-proANP (anti-canine NT-proANP (32-40) antibody), or the anti-canine NT-proANP (68-98) antibody may be a polyclonal antibody or a monoclonal antibody, each recognizing the amino acid sequence 74 to 82 of the canine NT-proANP (anti-canine NT-proANP (74-82) antibody).

In a further preferred embodiment of the present invention, the monoclonal antibody recognizing the amino acid sequence 32 to 40 of the canine NT-proANP (anti-canine NT-proANP (32-40) antibody) is assigned clone number 2E3 (Deposit No.: NITE P-1318) and the monoclonal antibody recognizing the amino acid sequence 74 to 82 of the canine NT-proANP (anti-canine NT-proANP (74-82) antibody) is assigned clone number 3D2 (Deposit No.: NITE P-1319).

In another aspect, the present invention provides the immunological measurement method for immunologically measuring the canine NT-proANP of non-human mammals such as dogs using the anti-canine NT-proANP antibody.

In a preferred embodiment, the present invention provides the immunological measurement method in which the anti-canine NT-proANP antibody recognizes a partial portion or a whole portion of the amino acid sequence 31 to 67 or the amino acid sequence 68 to 98 of the canine NT-proANP.

In a still further preferred embodiment of the present invention, the anti-canine NT-proANP (31-67) antibody may be a polyclonal antibody or a monoclonal antibody, each recognizing the amino acid sequence 32 to 40 of the canine NT-proANP (anti-canine NT-proANP (32-40) antibody) or the anti-canine NT-proANP (68-98) antibody may be a polyclonal antibody or a monoclonal antibody, each recognizing the amino acid sequence 74 to 82 of the canine NT-proANP (anti-canine NT-proANP (74-82) antibody).

In another preferred embodiment, the present invention provides the immunological measurement method selected form radioimmunoassay, enzyme immunoassay, luminescence immunoassay, and coagulation assay. In a more preferred embodiment of the present invention, the enzyme immunoassay may be selected from ELISA or immunochromatography assay, the coagulation assay from latex coagulation assay, and luminescence immunoassay from fluorescence immunoassay or chemiluminescence immunoassay.

In a still further aspect, the present invention provides an immunologically measuring kit for immunologically measuring the canine NT-proANP using the anti-canine NT-proANP antibody in accordance with the immunological measurement method of the present invention, which may be selected from radioimmunoassay, enzyme immunoassay, coagulation assay, and luminescence immunoassay, preferably enzyme immunoassay such as ELISA or immunochromatography assay.

In a still further aspect, the present invention provides an infection detection method for detecting heart diseases of a non-human mammal such as a dog, including mitral valve insufficiency, heart failure, etc., using the anti-canine NT-proANP antibody and the immunological measurement method according to the present invention.

In another preferred embodiment of this aspect, the present invention provides the infection detection method for detecting an infection infecting the heart of a non-human mammal such as a dog, including infectious diseases such as filariasis, by measuring the canine NT-proANP with the anti-canine NT-proANP antibody by the immunological measurement method of the present invention.

In a still further aspect, the present invention provides an immunochromatographic means for immunologically measuring canine NT-proANP in a sample of a non-human mammal such as a dog, which comprises an immunochromatographic support member, and a first antibody immobilized on colored particles, and a second antibody, wherein the first and second antibodies are each a member selected from an anti-canine NT-proANP (31-67) antibody recognizing amino acid sequence 31 to 67 of canine NT-proANP and an anti-canine NT-proANP (68-98) antibody recognizing amino acid sequence 68 to 98 thereof, provided however that when the first antibody is the anti-canine NT-proANP (31-67) antibody, the second antibody is the anti-canine NT-proANP (68-98) antibody, and vice versa.

In another preferred embodiment of the present invention, the immunochromatographic support member comprises an immunochromatographic strip for detecting the canine NT-proANP in the sample by visualizing the canine NT-proANP immobilizing the colored particles conjugated to the first antibody, which is further conjugated with the second antibody to detect the canine NT-proANP in the sample.

In a further preferred embodiment of the present invention, the immunochromatographic strip is provided with a sample pad, a conjugate pad, and a migration membrane with a test line, wherein the conjugate pad coated with the first antibody labeled with the colored particles conjugates the canine NT-proANP in the sample with the antibody labeled with the colored particles, and the test line of the migration membrane with the second antibody immobilized thereon further conjugates the second antibody with the canine NT-proANP in the sample migrated through the migration membrane, resulting in changing color of the test line to detect the presence of the canine NT-proANP in the sample.

In a still further preferred embodiment of the present invention, the anti-canine NT-proANP (31-67) antibody comprises an anti-canine NT-proANP (32-40) antibody recognizing the amino acid sequence 32 to 40 of canine NT-proANP (anti-canine NT-proANP (32-40) antibody), and the anti-NT-proANP (68-98) antibody comprises an anti-canine NT-proANP (74-82) antibody recognizing the amino acid sequence 68 to 98 of the canine NT-proANP (anti-canine NT-proANP (74-82) antibody).

As described above, the present invention provides the antibody recognizing a particular portion of the amino acid sequence of the canine NT-proANP different from that of the human NT-proANP, preferably antibodies recognizing the different portions (amino acid sequence 31 to 67 and amino acid sequence 68 to 98) of the amino acid sequence of the canine NT-proANP, the immunological measurement method for immunologically measuring the canine NT-proANP using the antibody, and the detection method for detecting heart diseases or infections infecting the heart of companion animals such as dogs, including mitral valve insufficiency, heart failure, or filariasis. As the present invention can specifically measure and detect the canine NT-proANP, the present invention has the effects of specifically and rapidly detecting heart diseases and infectious diseases of companion animals such as dogs, using the anti-canine NT-proANP specific antibody and a standard canine NT-proANP product, unlike conventional methods using the human NT-proANP.

In the present invention, when the immunochromatography assay is particularly used as the enzyme immunoassay, it can be conducted by very simple operation and for a comparably short time, and expensive devices are not required and may be hand-carried. Clinical application is greatly expected.

### BRIEF DESCRIPTION OF THE ACCOMPANYING DRAWINGS

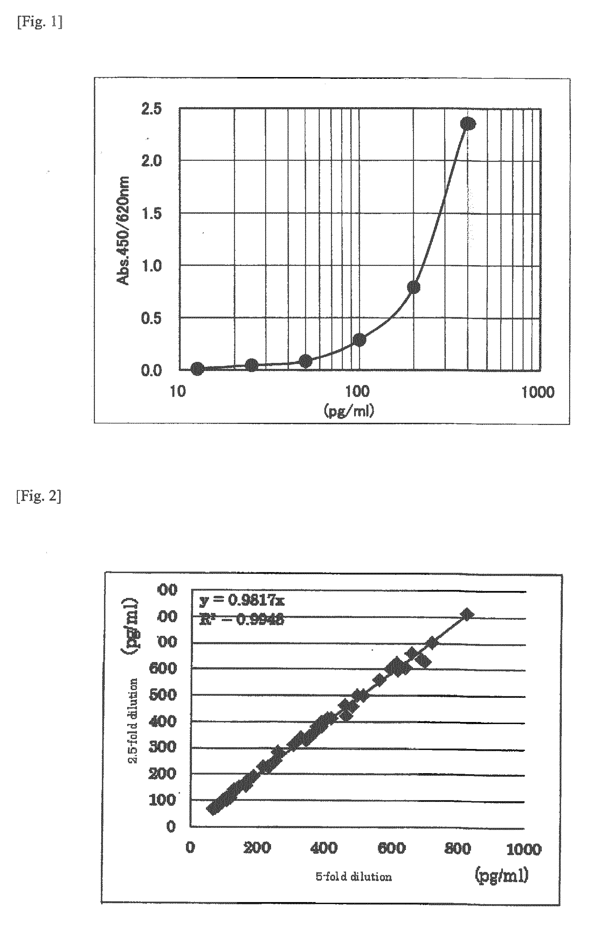

Fig. 1 is a view showing an example of a standard curve of canine NT-proANP and a measurement scope of the standard curve (12.5 - 400 pg/ml).
Fig. 2 is a view showing the correlation test of dilution ratios of samples and the results of the correlation test with 2.5-fold and 5-fold dilutions of blood samples from 53 dogs (beagles, Cavaliers, Chihuahuas, spitzs, Golden retrievers, and crossbreeds) diagnosed as canine heart failure.
Fig. 3 is a plan view showing an immunologically measuring kit of the present invention on the basis of an immunochromatographic assay.
Fig. 4 is a sectional view showing an immunologically measuring kit of the present invention on the basis of an immunochromatographic assay.
Fig. 5 is a view showing the results of measurement kit using the immunochromatographic kit.

### MODES FOR CARRYING OUT THE INVENTION

The present invention relates to an anti-canine NT-proANP antibody capable of recognizing a canine N-terminal pro-atrial natriuretic peptide (canine NT-proANP), an immunological measurement method for immunologically measuring the canine NT-proANP using the anti-canine NT-proANP antibody, as well as a method for detecting heart diseases or infectious diseases of companion animals such as dogs, etc., selected from mitral valve insufficiency or filariasis using the immunological measurement method.

The anti-canine NT-proANP antibody according to the present invention comprises an antibody capable of recognizing a partial portion or a whole portion of the amino acid sequence of the canine NT-proANP different from that of human NT-proANP. More specifically, the present inventions may include, but not be limited to, the antibody recognizing a partial portion or a whole portion of amino acid sequence 31 to 67 of the canine NT-proANP (hereinafter referred to as "anti-canine NT-proANP (31-67) antibody") and a partial portion or a whole portion of amino acid sequence 68 to 98 of the canine NT-proANP (hereinafter referred to as "anti-canine NT-proANP (68-98) antibody").

More specifically, the anti-canine NT-proANP (31-67) antibody may be a polyclonal antibody or a monoclonal antibody recognizing a partial portion or a whole portion of amino acid sequence 31 to 67 of the canine NT-proANP, which may include, for example, an antibody recognizing amino acid sequence 32 to 40 (SQ ID NO:2: AESPQALSE), although the monoclonal antibody is preferred. Hydridoma cells capable of producing the monoclonal antibody of the anti-canine NT-proANP (31-67) antibody are deposited as NITE P-1318 at NITE Patent Microorganisms Depository on May 9, 2012.

The anti-canine NT-proANP (68-98) antibody may be a polyclonal antibody or a monoclonal antibody recognizing a partial portion of amino acid sequence 68 to 98 of the canine NT-proANP, which may include, for example, an antibody recognizing amino acid sequence 74 to 82 (SQ ID NO:3: RSPWDSSDR), although the monoclonal antibody is preferred. Hydridoma cells capable of producing the monoclonal antibody of the anti-canine NT-proANP (68-98) antibody are deposited as NITE P-1319 at NITE Patent Microorganisms Depository on May 9,2012.

In accordance with the present invention, the antibodies recognizing the canine NT-proANP may be produced by conventional procedures known per se in the art involved.

As an antigen to be used for the production of the antibodies according to the present invention, there may be used, for example, a peptide having a particular portion of the amino acid sequence of the canine NT-proANP and a derivative thereof as well as a synthetic peptide having such a particular portion thereof. The peptide or the derivative thereof may be produced by conventional processes from tissues or cells of mammals such as mice, rats, etc. The synthetic peptides may be prepared, for example, by conventional chemical synthesis by peptide synthesizers or by incubation of transformants containing DNA coding for the peptide or the derivatives.

The antigens as produced above may be used for direct immunization in a solubilized form or for immunization as a complex conjugated with an appropriate carrier. As the carrier, there may be used, for example, a natural polymer carrier or a synthetic polymer carrier. The natural polymer carrier may include, for example, serum albumin, thyroglobulin, hemoglobin or hemocyanin of mammals such as cattle and the synthetic polymer carrier may include, for example, latex of polymers or copolymers, such as polyamino acids, polystyrenes, and polyacryls. For conjugating the antigen to the carrier, there may be used condensed compounds including, for example, diazonium compounds, dialdehyde compounds, dimaleimide compounds, maleimide-activated ester compounds, and carbodiimide compounds,.

The antigen as obtained above may be administered intraperitoneally, intravenously or subcutaneously as an immunogen for immunization to mammals such as mice, rats, rabbits, etc., to produce an antibody in the mammalian bodies. In order to enhance the ability of the production of antibodies, the immunogen may be emulsified in an appropriate adjuvant such as complete Freund's adjuvant or Freund's incomplete adjuvant, and the resulting emulsion may be administered to mammals. The immunization may be performed usually once per two to six weeks, followed by a total of two times to ten times of additional immunizations.

For the production of the antibodies according to the present invention, mammals such as mice are immunized with the antigen and the individuals having a recognized antibody value are selected from the antigen-immunized mammals, and the spleen is removed from the individual in two to five days after the final immunization. The antigen-producing cells (spleen cells) are then fused with myeloma cells derived from mammals such as mice to produce hybridoma strain producing the antibody of question according to the present invention. The fusion of the spleen cells with the myeloma cells may be performed using a fusion promoter such as polyethylene glycol by conventional procedures commonly used in this art. From the hybridoma strain obtained by the fusion, the hybridoma strain of question may be selected by screening or cloning. The resulting antibodies may be separated and purified, for example, by separation and purification procedures for immunoglobulin, such as salting-out, affinity chromatography, ion exchange column method, or gel filtration.

The antibodies according to the present invention may be produced by intraperitoneally administering the hybridoma strain to the mammals such as mice and collecting body fluids such as abdominal dropsy. They may be purified readily by conventional procedures as well known in the art.

The immunological measurement method according to the present invention may comprise, for example, the measurement method using the antibody having a potent affinity and a high specificity as a connecting reagent. The immunological measurement method of the present invention may include, for example, radioimmunoassay, enzyme immunoassay such as ELISA or immunochromatography assay, coagulation assay such as latex coagulation assay, luminescence immunoassay such as fluorescence immunoassay or chemiluminescence immunoassay. The measurement method to be used for the present invention is not limited to a particular one as long as the antibody of the present invention can be used therefor, although the enzyme immunoassay such as ELISA and immunochromatography is preferred. Therefore, the present invention will be described by taking the ELISA or immunochromatography as an example of the immunological measurement method of the present invention, however, it is understood as a matter of course that the present invention is not limited in any respect to the examples as will be described below.

It can also be noted herein that the immunological measurement method of the present invention may include a variety of variants including, for example, direct method, indirect method, competitive method, non-competitive method, and sandwich method. Among these methods, the sandwich method is preferred. These immunological measurement methods may be carried out each utilizing conventional measurement principles well known in this art.

The immunological measurement method according to the present invention may usually comprise a step of immobilizing an antibody (a primary antibody) on a support body such as a 96-well plate, beads such as polystyrene beads, a tube, or a membrane such as nitrocellulose membrane and incubating the primary antibody with a sample such as the blood or serum containing the antigen to be measured, a step of further incubating the resulting sample with another antibody (a secondary antibody) labeled with an enzyme and forming the enzyme-labeled antibody-antigen conjugate, and a step of measuring the antigen of question by detecting the resulting enzyme-labeled antibody-antigen conjugate. These steps may be carried out by conventional procedures as are well known in the art, except for the antibodies to be used. For the reagents to be used in these steps, conventional reagents may be used.

As the primary antibody to used in the immunological measurement method of the present invention, there may be used either of the anti-canine NT-proANP (31-67) antibody (2E3) recognizing the amino acid sequence 31 to 67 or the anti-canine NT-proANP (68-98) antibody (3D2) recognizing the amino acid sequence 68 to 98 of the canine NT-proANP. Either of the anti-canine NT-proANP (31-67) antibody or the anti-canine NT-proANP (68-98) antibody may also be used as a secondary antibody by labeling with an enzyme, a fluorescent substance, a luminescent substance, a radioactive isotope or coloring particles. For the conjugation of the antibody to the labeled substance, a compound of a biotin-avidin type may be used.

The enzymes to be used for labelling the antibodies in the present invention may include, for example, horseradish peroxidase (HRP), β-galactosidase (β-GAL), or alkali phosphatase (ALP). Fluorescent substances may include, for example, fluorescein, fluorescamine or fluorescein isothiocyanate. Luminescence substances may include, for example, luciferin, luminol or luminol derivatives. Radioactive isotopes may include, for example, [¹²⁵I], [¹³¹I], [³H], or [¹⁴C]. The coloring particles may include, for example, latex coloring particles of organic polymers such as polystyrenes, styrenes, or styrene-butadiene copolymers, or metal particles such as metal colloids, e.g., gold colloid or silver colloid, or metal sulfides.

As a specific example of a mode of the representative enzyme immunoassay, ELISA will be briefly described below in a generic term. It should be understood, however, that the present invention is not intended whatsoever to be limited to this example, and this is illustrated solely for the purpose to specifically describe the ELISA.

The enzyme-linked immunosorbent assay (ELISA) generally comprises binding an antigen as a substance for the object of measurement in a sample with a primary antibody (or a capture antibody); reacting the antigen captured by the primary antibody with a secondary antibody labeled with an enzyme to form a conjugate of the antigen with the enzyme-labeled secondary antibody; further reacting the resulting antigen-secondary antibody-enzyme conjugate with a chromogenic substrate to convert the substrate into a pigment via the action of the enzyme; and measuring an absorbance of the pigment to quantitatively identify the antigen of question.

More specifically, first, the primary antibody is coated or immobilized, for example, on the surfaces of the wells of a 96-well microplate as a support member in order to capture a substance as the object of measurement. To the wells of the microplate, a sample or a standard solution containing the substance as an antigen of question is then added to cause the antigen to be conjugated to the primary antibody coated or immobilized on the wells of the microplate to form an antigen-primary antibody complex.

Next, a secondary antibody labeled with an enzyme is added to the wells and reacted with the antigen-primary antibody conjugate coated or immobilized thereon to form a conjugate of the enzyme-labeled secondary antibody with the antigen-primary antibody complex. The secondary antibody may be chemically labeled with an enzyme to form an enzyme-labeled secondary antibody recognizing an epitope different from the epitope that is recognized by the primary antibody or capture antibody.

Thereafter, a chromogenic substrate solution may be added to convert the chromogenic substrate into a pigment by means of the reaction with the enzyme of the enzyme-labeled secondary antibody. The enzyme reaction is then terminated with a quenching agent including an acid such as sulfuric acid. After the termination of the enzyme reaction, the antigen connected to the capture antibody may be measured by measuring the absorbance by means of measurement for the coloration of the pigment by the colorimetric method. The amount of the antigen in the sample may be computed from the absorbance. As described above, it is preferred to carry out the sandwich ELISA using a three-component sandwich system, that is, an antibody-antigen-antibody sandwich system.

In the embodiment as described above, the enzyme-labeled secondary antibody is used. It should be noted herein, however, that on labeling the antibody with the enzyme, this antibody may be caused to undergo steric hindrance on the connecting ability of the antibody, thereby making it difficult to allow a number of enzymes to be connected to the antibody of one molecule. In order to overcome such difficulty, accordingly, a biotin-labeled second antibody may be preferably used. Then, an avidin-enzyme conjugate with the enzyme connected to avidin, which is contained in the egg white, may be preferably reacted with the biotin-labeled secondary antibody connected to the antigen. As the avidin-enzyme complex, there may be used, for example, avidin-peroxidase complex, in which horseradish peroxidase (HRP) may be preferably used as the peroxidase. As the chromogenic substrate for the avidin-enzyme complex such as avidin-peroxidase complex, there may be used, for example, TMB (3,3',5,5'-tetramethylbenzidine). After the reaction with the chromogenic substrate, the reaction is terminated with a reaction terminator such as an acid, e.g., sulfuric acid, followed by measuring the absorbance of the resulting pigment by conventional measuring procedures well known in the art.

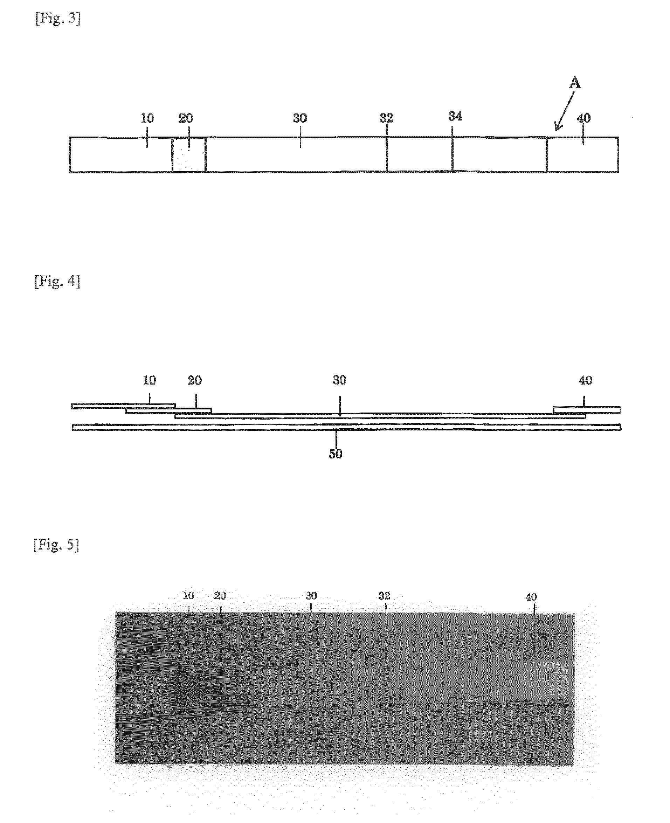

As another representative enzyme immunoassay among those immunoassays as described above, the immunochromatographic assay will be briefly described with reference to Figs. 3 and 4. Figs. 3 and 4 are a plan view and a sectional view showing a chromatographic strip as an example, respectively, which may be used for the immunological assay kit on the basis of the immunochromatography assay of the present invention.

As shown in Figs. 3 and 4, the immunochromatographic strip (A) may generally comprise a sample pad 10 disposed at one end of the strip, a conjugate pad 20 disposed under the sample pad 10 to allow a lateral flow of the sample from the sample pad to the conjugate pad. Underneath the conjugate pad, a migration membrane 30 is disposed extending in the downstream direction up to an absorbent pad 40 disposed at the other end of the strip so as for the sample migrated from the conjugate pad to laterally flow in and through the migration membrane through a test line 32 to a control line 34. Underneath the migration membrane 30, a backing sheet 50 is disposed supporting the entire length of the strip (A). The immunochromatographic strip (A) may be structured in such a manner that the sample poured on the sample pad 10 flows laterally and continuously by the capillary action of the structuring materials through the conjugate pad 20 and the migration membrane 30 to the absorbent pad 40.

The sample pad 10 is the place onto which the sample is poured and which may be made of a raw material having a homogenous quality and having the physical property of laterally flowing, that is, permeating and developing the sample in and through the material by means of its capillary action. The raw material to be used for the sample pad may include, for example, cellulose fibers, glass fibers, polyurethanes, polyacetates, acetate celluloses, and nylons. As the sample to be measured by the present invention, there may be mentioned fluid samples including, for example, body liquids of mammals such as blood, serum and plasma and excretions such as urine.

The conjugate pad 20 is to hold a labeled antibody, which may be made of raw materials including, for example, cellulose fibers, glass fibers or non-woven cloth. The conjugate pad 20 may be prepared by dropwise pouring or impregnating a given amount of the labeled antibody into the above material and drying the material. At the conjugate pad 20, the antigen contained in the sample migrated from the sample pad may be conjugated with the labeled antibody held on the conjugate pad 20, followed by capturing and recognizing the antigen in the form of a labeled antibody-antigen complex.

The labeled antibody to be held in the conjugate pad 20 may be prepared by labeling the antibody with a labeling substance which may preferably include, for example, colloidal metal particles such as colloidal gold particles or colloidal silver particles or composite colloidal particles. Average particle sizes of such colloidal metal particles may be in the range from approximately 1 to 500 nm, preferably from approximately 1 to 50 nm. The labeling reaction of the antibody by the coloring particles may be performed in accordance with conventional procedures well known in the art.

The migration membrane 30 plays a role as a chromatographic carrier and may be preferably in a sheet form made of porous material including, for example, nitrocellulose membrane, cellulose membrane, nylon membrane, glass fibers or nonwoven cloth. The materials to be used for the membrane are not limited to a particular one as long as they are able to develop and migrate the sample in and through the membrane by means of the capillary action. The migration membrane 30 is structured so as to allow the sample in the conjugate pad 20 to laterally flow through the membrane toward the adsorbent pad 40 by the capillary action.

The migration membrane 30 may be provided with a test line 32 on the downstream side in the direction in which the sample laterally flows and develops. The test line 32 works as a zone for deciding the presence (positive) or absence (negative) of the substance of question in the tested sample. The test line may be immobilized with an antibody different from the labeled antibody held on the conjugate pad 20 in order for the labeled antibody to be captured on the test line. If the labeled antibody held on the conjugate pad 20 would be the primary antibody, the secondary antibody is immobilized on the membrane. On the contrary, if the labeled antibody held on the conjugate pad would be the secondary antibody, the primary antibody is immobilized on the membrane.

As the primary antibody, there may be used the anti-canine NT-proANP (31-67) antibody or the anti-canine NT-proANP (68-98) antibody. As the secondary antibody, there may also be used the anti-canine NT-proANP (31-67) antibody or the anti-canine NT-proANP (68-98) antibody. It is provided, however, that, in the event that one of the two antibodies is used as the primary antibody, then the other is used as the secondary antibody.

At the test line 32 of the migration membrane 30, the immobilized second antibody may capture the labeled antibody-antigen complex, which may be in turn recognized in the form of a three-component sandwich system, i.e., labeled antibody-antigen-antibody sandwiched conjugate system, by means of the antigen-antibody reaction. As a result, if the substance as the object of measurement would be contained in the sample measured, it may then be captured and recognized in the tree-component sandwich conjugate system and visualized by the coloring particles which are used for the labeling agent of the labeled antibody. By using the colloidal metal particles or other substances as the labeling agent as described above, the presence or absence of the antigen of question in the sample tested may be visualized, resulting in realizing precise and simple measurement and an extremely useful tool for measurement on site.

The migration membrane 30 may also be provided with a control line 34 on the downstream side of the test line 32 and immobilized with an antibody for the labeled antibody, thereby allowing the labeled antibody to be captured by the antigen-antibody reaction and visualized. Even if the labeled antibody would be decided to be negative, only the control line 34 is visualized by the coloring particles.

An absorbent pad 40 is a pad for absorbing the sample which was added to the sample pad and then was migrating through the conjugate pad and the migration membrane by means of chromatography. The absorbent pad may be made of a highly absorptive material such as filtering paper. A backing sheet 50 may be disposed extending from the lower end of the sample pad 10 up to the lower end of the absorbent pad 40 supporting the entire length of the strip (A). A material for the backing pad 50 is not limited to a particular material.

The immunochromatography assay using the immunochromatographic strip (A) will be briefly described as follows with reference to Figs. 3 and 4. A sample containing a substance (an antigen) to be measured is dropwise poured onto a sample inlet of the sample pad 10 and flows laterally, developing and migrating to the conjugate pad 20. In the conjugate pad 20, the sample containing the antigen of question flows laterally through the material, resulting in conjugating with the labeled antibody labeled with the labeling substance and forming an antigen-labeled antibody complex by means of the antigen-antibody reaction during the lateral flow in the conjugate pad. After the sample containing the two-component complex, i.e., antigen-labeled antibody complex, develops and migrates through the conjugate pad, it reaches the migration membrane 30 and further develops and migrate therethrough up to the test line 32. In the test line, the antigen in the form of the antigen-labeled antibody is then conjugated with the antibody immobilized on the test line, forming a three-component sandwiched conjugate system, i.e., a labeled antibody-antigen-immobilized antibody sandwiched conjugate system, which is in turn visualized with the labeling substance such as the colloidal metal particles, etc. Therefore, in the event that the substance of question is contained in the sample, the test line is visualized and decided to be positive. On the other hand, if the labeled antibody would be determined as negative, only the control line 34 is visualized by the coloring particles. The sample further develops and migrates to the adsorbent pad 40 where the sample is absorbed.

The immunologically measuring kit according to the present invention may comprise at least a support body such as a 96-well microplate or an immunochromatographic strip, a first antibody (anti-canine NT-proANP (31-67) antibody (2E3)), a second antibody (anti-canine NT-proANP (68-98) antibody (3D2)), and a labeling substance such as an enzyme or colloidal metal.

When the immunologically measuring kit of the present invention is used as an enzyme-linked immunosorbent assay (ELISA) kit, the immunologically measuring kit may comprise a 96-well microplate as the support body, HRP or an enzyme complex composed of biotin, avidin and HRP as the labeling substance, TMB as the chromogenic substrate, and an acid such as sulfuric acid as the quenching agent.

When the immunologically measuring kit of the present invention is used as an immunochromatographic assay kit, the immunochromatographic strip may be used as a support body. On the immunochromatographic strip, it is preferred that the conjugate pad may be immobilized with the labeled antibody labeled with the labeling substance such as colloidal metal particles, including a labeled first antibody (anti-canine NT-proANP (31-67) antibody (2E3)) or a labeled second antibody (anti-canine NT-proANP (68-98) antibody (3D2)), and the test line of the membrane may be immobilized with the antibody different from the antibody immobilized on the conjugate pad, that is, the first or second antibody. The immunochromatographic assay kit may contain a developing solution for developing the sample, in addition to the immunochromatographic strip. The immunochromatographic assay kit is convenient for handling in use on site.

The present invention will be described in more detail by way of examples. It is to be understood, however, that those examples are described in no way whatsoever to limit the present invention and intended to be described solely for illustrative purposes to describe the present invention in a more specific fashion. Therefore, the preferred embodiments contained in those examples are meant to be encompassed within the scope of the present invention.

### EXAMPLE 1

### (Preparation of Immunogen)

An immunogen was prepared in the following way in order to prepare hybridoma strain producing a monoclonal antibody.

As an antigen, there were prepared a synthetic peptide #1 (CAESPQALSE) by adding cysteine to the N-terminal side of the amino acids 32 to 40 of the amino acid sequence of canine NT-proANP and a synthetic peptide #2 (RSPWDSSDRC) by adding cysteine to the C-terminal side of the amino acids 74 to 82 of the amino acid sequence of canine NT-proANP. Then, each of the synthetic peptides was connected to a carrier protein (KLH: keyhole limpet hemocyanin) using Imject maleimde-activated JKH kit (Pierce Cat #77606).

More specifically, distilled water was added to Imject maleimide mcKLH reagent to make 10 mg/ml. To 6 mg/ml of maleimide-activated mcKLH was added 3.0 mg of the synthetic peptide dissolved in a buffer solution of the kit to make 3.0 ml. After the reaction at 25°C for 2 hours, the reaction mixture was dialyzed at 4°C for two nights against saline solution to yield a synthetic peptide-KLH conjugates (each 7 mg). This was used as the immunogen.

The entire amino acid sequence of canine NT-proANP (consisting of 98 amino acids) is as described below:
SQ ID NO:1 
NPVYGSVSNA DLLDFKNLLD RLEDKMPLED EAESPQALSE QNAEAGAALS
PLPEVPPWTG EVSPAQRDGG ALGRSPWDSS DRSALLKSKL RALLAAPR
The molecular weight of the canine NT-proANP is determined to be 10,468 from the amino acid composition.

The following is the amino acid sequence of canine NT-proANP synthetic peptide (consisting of 98 amino acids) as a standard product of the canine NT-proANP measurement system:

NPVYGSVSNA DLLDFKNLLD RLEDKMPLED EAESPQALSE QNAEAGAALS
PLPEVPPWTG EVSPAQRDGG ALGRSPWDSS DRSALLKSKL RALLAAPR

For reference, the amino acid sequence 32 to 40 of the human NT-proANP is represented by VVPPQVLSE, and the amino acid sequence 74 to 82 of the human NT-proANP is represented by RGPWDSSDR.

Two kinds of the immunogens as prepared by the same processes were immunized in substantially the same manner as described above. BALB/c mice (Nippon SLC KK) were immunized by intraperitoneally administering 100 µg per mouse of the above synthetic peptide-KLH conjugate. After the initial immunization, additional immunization was conducted three times at the interval of two weeks. For the initial and second immunization, complete Freund's adjuvant was administered.

### (Production of Hybridoma strain producing monoclonal antibodies)

Two kinds of the hybridoma strains producing monoclonal antibodies were produced by the same processes. The spleen cells (1.1 x 10⁸ cells) were removed from the immunized mouse at day 3 after the final immunization and fused with mouse myeloma cells (P3-X63-Ag8.653; DS Pharma Biomedical K.K.; 2.1 x 10⁷ cells) using 50% polyethylene glycol 4000 (Merck). The fused hybridoma strain was selected in a medium containing hypoxanthine, aminopterin and thymidine. In day 10 after the cell fusion, a screening was conducted by ELISA for the hybridoma strain producing a specific antibody.

The ELISA was carried out in the following way. To each well of a 96-well microplate (Nunc) was added 100 µl of PBS (pH 7.4) containing 0.15 M NaCl and 10 µg/ml of the synthetic peptide #1 (CAESPQALSE) or the synthetic peptide #2 (RSPWDSSDRC), and it was immobilized overnight at 4°C. Then, the well was washed once with a solution containing 25% BlockAce (DS Pharma Biomedical K.K.), and the same solution (200 µl) was then added to block the reaction. Thereafter, each well was washed three times with PBS containing 0.05% Tween 20 (registered trademark), followed by addition of 100 µl of a supernatant of the hybridoma cell culture and reaction at room temperature for 2 hours. The well was then washed three times and 100 µl of PBS containing 1 µg/ml of HRP-labeled anti-mouse IgG was added, followed by reaction at room temperature for 2 hours and washing three times. Then, 100 µl of TMB (3,3',5,5'-tetramethylbenzidine) (Dako) was added and the resulting mixture was then subjected to enzyme reaction at room temperature for 20 minutes, followed by addition of 100 µl of 1M sulfuric acid solution to suspend the reaction. After the suspension of the reaction, each well was measured for the absorbance at a main wavelength of 450 nm and an auxiliary wavelength of 620 nm with a plate reader (Teccan).

Further, the hybridoma strain indicating the positive production of the specific antibody were cloned three times by limiting dilution method, yielding hybridoma strain #1 and #2, producing the monoclonal antibodies recognizing the synthetic peptide #1 and the synthetic peptide #2, respectively. A culture supernatant of the resulting hybridoma strain #1 or #2 was determined with a mouse monoclonal antibody isotyping test kit (Serotec) for the subclass of the antibody produced by the hybridoma strain #1 and #2, respectively. The newly obtained monoclonal antibodies are as described below.
Hybridoma strain #1: Clone Number: 2E3; Isotype: IgG1 (κ)
Hybridoma strain #2: Clone Number; 3D2; Isotype; IgG1 (κ)

As a result of confirmation of the reactivity with the monoclonal antibodies 2E3 and 3D2 by the ELISA, it was confirmed that the monoclonal antibody 2E3 reacted with the synthetic peptide #1 but not with the synthetic peptide #2, while the monoclonal antibody 2E3 reacted with the synthetic peptide #2 but not with the synthetic peptide #1. It is thus confirmed that the two antibodies recognize the different portions of the canine NT-proANP containing both peptides by the fact that the antibody 2E3 recognizes the synthetic peptide #1 while the antibody 3D2 recognizes the synthetic peptide #2.

The hybridoma strain (clone number: 2E3) producing the monoclonal antibody #1 was deposited as NITE P-1318 at NITE Patent Microorganisms Depository on May 9, 2012. The hybridoma strain (clone number: 3D2) producing the monoclonal antibody #2 was deposited as NITE P-1319 at NITE Patent Microorganisms Depository on May 9,2012.

### EXAMPLE 2

### (Procedures for Immunological Measurement)

The anti-canine NT-proANP (31-67) antibody (clone number: 2E3) was immobilized on each well of a 96-well microplate by conventional procedures and then washed three times with 300 µl/well of a washing solution. A diluted sample (10 µl of plasma diluted by five times) or a standard solution (synthetic canine NT-proANP peptide containing a stabilizer) was added at the rate of 50 µl/well, and the microplate was stirred three times at 800 rpm and room temperature for 10 seconds. The mixture was then allowed to stand for 2 hours and washed three times with 300 µl/well of the washing solution. After washing, 50 µl/well of biotin-labeled anti-canine NT-proANP (68-98) antibody (clone number: 3D2) was added to each well of the microplate, followed by stirring three times at 800 rpm at room temperature for 10 seconds. After the microplate was left to stand for 2 hours, each well was washed three times with 300 µl/well of the washing solution, and 50 µl/well of peroxidase-avidin conjugate was added to each well, followed by stirring the mixture three times at 800 rpm and room temperature for 10 seconds and leaving it to stand for 30 minutes. Thereafter, 50 µl/well of TMB (3,3',5,5'-tetramethylbenzidine) was added, and the mixture was stirred three times at 800 rpm at room temperature for 10 seconds, followed by leaving it to stand for 30 minutes and terminating the reaction by adding 50 µl of 1M sulfuric acid to each well. After stirring, the absorbance (having a main wavelength of 450 nm and an auxiliary wavelength of 620 nm) was measured.

### EXAMPLE 3

Samples collected from healthy dogs and dogs with mitral valve insufficiency were measured for canine NT-proANP in accordance with the procedures for immunological measurement as described in Example 2 above. The results are shown in Tables 1 and 2 below.

In this Example, healthy blood samples were collected from 9-12-month-old healthy beagles under blood collection conditions using EDTA-2Na (final concentration: 1.0 - 1.5 mg/ml) and aprotinin (final concentration; 100 - 500 KIU/ml; KIU: Kallikrein Inhibitor Unit). The above blood samples were used as samples of healthy dogs.

On the other hand, blood samples were collected from dogs with mitral valve insufficiency (MR) under the same blood collection conditions as used for the healthy beagles. The dogs used for test were 4-13-years-old dogs (Cavaliers, Chihuahuas, Shih Tzu, spitzs, beagles, and yorkies).

**Table 1:**

| Healthy Dogs | Measured Canine NT-proANP Values (pg/ml) |
|---|---|
| 1 | 55.5 |
| 2 | 73.2 |
| 3 | 46.6 |
| 4 | 43.6 |
| 5 | 95.5 |
| 6 | 66.8 |
| 7 | 50.3 |
| 8 | 55.3 |
| 9 | 87 |
| 10 | 107 |
| 11 | 127 |
| 12 | 33.4 |
| 13 | 83.7 |
| Average | 71.14615 |
| SD | 27.60865 |
| SE | 7.657262 |

**Table 2:**

| MR | Measured Canine NT-proANP Values (pg/ml) |
|---|---|
| MR1 | 447 |
| MR2 | 575 |
| MR3 | 531 |
| MR4 | 370 |
| MR5 | 565 |
| MR6 | 588 |
| MR7 | 793 |
| MR8 | 468 |
| MR9 | 544 |
| MR10 | 698 |
| MR11 | 391 |
| MR12 | 497 |
| MR13 | 459 |
| MR14 | 614 |
| MR15 | 719 |
| MR16 | 556 |
| Average | 551 |
| SD | 116.1685 |
| SE | 29.04213 |

From the above results of measurement, the canine NT-proANP values of the dogs with mitral valve insufficiency (MR) were found to be significantly higher than those of the healthy dogs at p<0.01 by the t-test.

### EXAMPLE 4

Blood samples (HWD) were collected from 8-14-years-old dogs (spitzs, Labradors, crossbreeds, and golden retrievers) with filariasis under the same blood collection conditions as used in Example 3. The results of measurement are shown in Table 3 below.

**Table 3**

| HWD | Measured Canine NT-proANP Values (pg/ml) |
|---|---|
| HWD1 | 1190 |
| HWD2 | 745 |
| HWD3 | 421 |
| HWD4 | 657 |
| HWD5 | 617 |
| HWD6 | 637 |
| Average | 711 |
| SD | 257.6528 |
| SE | 105 |

From the above results of measurement, the canine NT-proANP values of the dogs with filariasis (HWD) were found to be significantly higher than those of the healthy dogs at p<0.01 by the t-test.

### EXAMPLE 5

Blood samples were collected from dogs diagnosed to be ISACHC Classification 1b and 2 as the degrees of cardiac diseases of dogs according to Dog's Cardiac Diseases by International Small Mammal Cardiac Health Council (ISACHC 1992). The blood collection was performed in accordance with the procedures for immunological measurement as described in Example 2, and the blood samples were measured in the same manner as used in Example 3. The results are shown in Table 4 below.

**Table 4**

| ISACHC | Measured Canine NT-proANP (pg/ml) |
|---|---|
| ISACHC 1b | 165 |
| ISACHC 1b | 234 |
| ISACHC 1b | 307 |
| ISACHC 2 | 561 |
| ISACHC 2 | 376 |
| ISACHC 2 | 824 |
| ISACHC 2 | 342 |
| ISACHC 2 | 390 |
| ISACHC 2 | 514 |
| ISACHC 2 | 698 |
| ISACHC 2 | 687 |
| Average | 463.45 |
| SD | 210.01 |
| SE | 63.32 |

### (Results of Measurement)

It is found that an average value of the results of measurement for all the dogs with heart diseases is 595 pg/ml, the SD value is 175 pg/ml, and the SE value is 37 pg/ml, when computed from a combination of the canine NT-proANP values of the dogs with mitral valve insufficiency (MR) as indicated in Table 2 above and those of the dogs with filariasis (HWD) as indicated in Table 3 above.

It is further found that an average value of the results of measurement for all the dogs with heart diseases is 551 pg/ml, the SD value is 195 pg/ml, and the SE value is 34 pg/ml, when computed from a combination of the results of measurement for the dogs with mitral valve insufficiency (MR) as indicated in Table 2 above, the dogs with filariasis (HWD) as indicated in Table 3 above, and the dogs with the classes of the cardiac diseases classified as the ISACHC Classification 1b and 2 as indicated in Table 4 above.

From those results as described above, the immunological measurement method for canine NT-proANP according to the present invention is found useful for physical examination of dogs particularly for mitral valve insufficiency and infections paralyzed in the heart, if it is assumed that dogs are determined as healthy when the measured canine NT-proANP values are within the scope (126.3 pg/ml, 2SD + average) of a healthy dog.

### EXAMPLE 6

This example is to investigate blood samples of dogs regarding heart diseases using commercially available human NT-proANP (1-98) ELISA kit (Bio Medica).

Blood samples were collected from healthy dogs and dogs with heart diseases under the blood collection procedures as described in Example 3 above and measured in accordance with the procedures of immunological measurement as described in Example 2, using the above kit. Table 5 shows the results of measurement for the blood samples from the healthy dogs and the dogs with heart diseases using the canine NT-proANP ELISA kit of the present invention and the human NT-proANP ELISA kit (Bio Medica).

**Table 5**

| | Times of Freezing & Thawing | Measuring Procedures | | | | | |
|---|---|---|---|---|---|---|---|
| | | Canine NT-proANP ELISA KIT (Invention) | | | Human NT-proANP ELIS KIT (Bio Medica) | | |
| | | Average | SD | CV | Average | SD | CD |
| | | (pg/ml) | (pg/ml) | (%) | (nmol/l) | (nmol/l) | (%) |
| Healthy Dogs | First | 77.6 | 13.6 | 17.6 | N.D. | | |
| | Second | 77.5 | 13.2 | 17.1 | N.D. | | |
| Dogs with Heart Disease | First | 208.7 | 206.0 | 98.7 | N.D.(11/12) * 1.01 | | |
| | Second | 198.3 | 204.0 | 102.9 | N.D.(11/12) * 0.971 | | |

In the table above, the symbol "N.D." means "Not Detected", i.e., not detected due to the detection limit or lower (< 6.59 ng/ml = < 0.63 nmol/l). The symbol "*" means measurement of one sample out of 12 samples. First time of freezing and thawing: 1.01 nmol/l; and second time of freezing and thawing: 0.971 nmol/l.

In this example, the blood samples were collected from healthy 9-12-months-old male beagles, while the blood samples were collected from 7-15-years-old dogs diagnosed as heart diseases (mitral valve insufficiency: 11 dogs; heart disease other than mitral valve insufficiency: 1 dog). The dogs were: miniature Dachshund, crossbreeds, yorkies, Shih Tzu, Cavaliers, Yorkshire terriers, Maltese, Welsh corgis, and spitzs.

In this Example, the blood samples were measured using the canine NT-proANP ELISA kit (according to the present invention) in accordance with the immunological measurement method as described in Example 2. The scope of measurement was: 12.5 - 400 pg/ml = 1.19 - 38.2 pmol/l.

On the other hand, the blood samples were similarly measured using the human NT-proANP (1-98) ELISA Kit (commercially available) in accordance with the immunological measurement method as described in Example 2. The scope of measurement was: 0.63 - 10 nmol/ml = 6.59 - 104.7 ng/ml (when computed as the molecular weight of canine NT-proANP: 10,467).

The measurement was carried out by a sandwich assay using a sheep-derived polyclonal anti-human NT-proANP (1-98) antibody as the antibody, and human NT-proANP (1-98) synthetic peptide as a standard product. The specificity was: human proANP (1-30) < 1%; human proANP (31-67) <1%; human proANP (79-98) < 1%, and ANP < 1%. The median value of the human sample was 1.45 nmol/l (as a reference value of blood plasma).

Each sample was pretreated by a confirmation test under storage conditions. The freezing and thawing at the first time were conducted by storing the sample at -35°C in the state in which the sample was obtained as it was and then thawing at the time of use for measurement. The freezing and thawing at the second time were conducted as a confirmation test as to confirm the influences on the values to be measured by the freezing and thawing operations under transportation conditions and at the time of re-examination. The samples were treated as follows: sample obtained (frozen at -35°C) → thawed at room temperature (22.8°C) for 30 minutes (measured #1) → stored at 4°C for 18 hours → frozen (-35°C) → thawed → measured #2.

### (Results of Measurement)

The results obtained by the measurement using the canine NT-proANP ELISA kit according to the present invention was found to be within the measurement scope of 12.5 to 400 pg/ml = 1.19 - 38.2 pmol/ml. The results obtained by the measurement using human NT-proANP (1-98) ELISA Kit (Bio Medica) revealed that the measured value of only one sample was within the measurement scope, while the measured values of all the other samples were outside the standard curve (lower values).

The commercially available standard kit product contained synthetic human NT-proANP (1-98) product, which has a measurement scope of 0.63 - 10 nmol/l (a reference value of human plasma: the median value of the human NT-proANP: 1.45 nmol/l) and a measurement scope (by weight) of 6.59 - 104.7 ng/ml.

As described above, the measurement scope of the commercially available product was found to be higher in concentration by from approximately 260 to 520 times than that of the canine NT-proANP (1-98) ELISA kit according to the present invention.

From the above test results, it is found that the reproducible results were obtained up to the second time of the freezing and thawing of the canine samples. A reference document (Inge, T., The Veterinary Journal, 180, 195-201, 2009) reveals that an average value of the measured values for control dogs was 246 pmol/l (2,570 pg/ml) by the commercially available human NT-proANP (1-98) ELISA Kit (Bio Medica). This indicates that this measurement scope of the human NT-proANP (1-98) ELISA Kit (Bio Medica) (0.63 - 10 nmol/l; the median value of human samples: 1.45 nmol/l) are lower than the detection limit for the healthy dog samples. The above results indicate that the measurement scope of the commercially available product is lower than the detection limit, too. Moreover, the average value of the measured values obtained using the human NT-proANP ELISA kit was found higher by approximately 36 times in concentration than the average value (71 pg/ml) of the values measured for the blood samples of the healthy dogs by the canine NT-proANP ELISA kit according to the present invention. These findings are considered that the commercially available sheep-derived polyclonal antibody recognizing human NT-proANP (1-98) is not matched with the specificity and the reactivity to the canine NT-proANP. Further, this is considered to indicate the necessity of a measurement system exclusive for measuring the canine NT-proANP by an anti-canine NT-proANP-specific antibody and a standard product.

### EXAMPLE 7

This example concerns the immunological measurement method by an immunochromatographic assay.

### Preparation of Materials:

### (Colloidal Gold Suspension)

Colloidal gold suspension was prepared by diluting colloidal gold (WINERED CHEMICAL CO. LTD.) by 2 times with 10 mM Tris (hydroxymethyl) aminomethane buffer (Tris buffer, Wako Pure Chemical Industries, Ltd., pH 8.6).

### (Antibody Solution)

An antibody solution was prepared by adjusting anti-canine NT-proANP (68-98) antibody (clone #3D2) to the concentration of 0.2 mg/ml with 10 mM Tris buffer (pH 8.6).

### (BSA-PEG Mixed Solution)

A 1% BSA-PEG mixed solution was prepared by dissolving 1 gram of BSA in 10 mM Tris buffer (pH 8.6) and increasing the volume to 100 ml to obtain 1% BSA-10 mM Tris buffer (pH 8.6) and mixing 4.5 ml of the resulting 1% BSA-10 mM Tris buffer (pH 8.6) with 0.5 ml of 1% PEG solution (PEG4000; Wako Pure Chemical Industries, Ltd.).

### (Storage Solution for Labeled Colloidal Gold)

A 5% D(+) trehalose dihydrate solution was prepared by dissolving 5 grams of D(+) trehalose dihydrate (Wako Pure Chemical Industries, Ltd.) in purified water and increasing the volume to 100 ml and then dissolving 1 gram of BSA in the resulting trehalose dihydrate solution, followed by increasing the volume to 100 ml to make a 1% BSA-5% D(+) trehalose dihydrate solution, which was used as a storage solution for the labeled colloidal gold.

### (Sample Migration Buffer)

A sample migration buffer was prepared by dissolving 2.5 g of casein sodium (Wako Pure Chemical Industries, Ltd.) in 10 mM Tris buffer (pH 8.6) and increasing the volume to 100 ml to make 2.5% casein sodium-10 mM Tris buffer (pH 8.6).

This sample migration buffer is mixed with a sample to be measured and allowed to develop in and through the strip and migrate therethrough. After dropwise addition to the sample pad of the strip, the sample migration buffer develops and migrate the antigen contained therein in and through the membrane of the strip.

### (Labeling Reaction)

A mixture of 100 µl of the colloidal gold suspension with 100 µl of an anti-canine NT-proANP (68-98) antibody (clone #3D2) solution was poured into a 0.5 ml siliconized tube and mixed by a Vortex mixer and left to stand for 15 minutes. The resulting suspension was then mixed with 200 µl of 1% BSA-PEG mixed solution and mixed by a Vortex mixer. After the suspension was left to stand for 15 minutes, it was then centrifuged at 8,000 g at 4°C for 3 minutes and the supernatant was then removed. To the remaining precipitate, 200 µl of 1 % BSA-PEG mixed solution was added and the resulting mixture was mixed with a Vortex mixer and left to stand for 15 minutes. After centrifugation at 8,000 g at 4°C for 3 minutes, the supernatant was removed. The remaining precipitate was then mixed with 100 µl of the storage solution for the labeled colloidal gold and the colloidal gold particles were allowed to disperse in the solution. The colloidal gold particles, the other reagents and the solution were stored at 2-8°C.

### Preparation of Materials for Immunochromatographic strip:

### (Conjugate Pad)

The conjugate pad was prepared by coating 50 µl of the colloidal gold-labeled antibody dispersion as prepared above on the tip portion of glass fiber (Glass Fiber Conjugate Pad from Millipore) and drying at room temperature.

### (Antibody-Immobilized Membrane)

As a material for the antibody-immobilized membrane, there was use a membrane (Hi Flow Plus HFB09004 from Millipore).

### (Dilution Buffer for IgG)

A dilution buffer for IgG was prepared by mixing 10 mg of casein with 5 ml of 10 mM phosphate buffer (Wako Pure Chemical Industries, Ltd, pH 7.4).

### (Antibody for Immobilization)

Mouse IgG-anti-canine NT-proANP (31-67) antibody (clone #2E3) was prepared by diluting the antibody with the IgG-dilution buffer to make 100 µg/ml.

### (Blocking Solution)

A blocking solution was prepared by dissolving 0.5 g of casein in 20 mM phosphate buffer (pH 7.4) and increasing the volume to 100 ml to make 0.5% casein-20 mM phosphate buffer (pH 7.4).

### (Sucrose Solution)

A 3% sucrose-0.3% cholic acid solution was prepared by dissolving 3 g of sucrose (Wako Pure Chemical Industries, Ltd) in 0.3% cholic acid solution (Wako Pure Chemical Industries, Ltd) and increasing the volume to 100 ml. This was used as a sucrose solution.

### (Other Materials)

Cellulose Fiber Sample Pad (Millipore) was used as a sample pad, Absobet Pad (Millipore) as an absorbent pad, and a backing sheet (Nippon Engineering Co., Ltd.) as a packing sheet.

### (Preparation of Immunochromatographic Strip)

The immunochromatographic strip used in this example was prepared using the material for the immunochromatographic strip as prepared above. First, 50 µl of colloidal gold labeled with the anti-canine NT-proANP (68-98) antibody (clone #3D2) was coated to the tip portion of the conjugate pad (20) and dried at room temperature. Then, 6 µl of anti-canine NT-proANP (31-67) antibody (clone #2E3) (100 µg/ml) was coated to the test line of 1 mm width of the membrane (30) as the immobilized antibody and dried for 60 minutes at room temperature. The membrane was then immersed in the blocking solution and shaken slowly for 20 minutes, followed by immersing in purified water and shaking slowly twice for 5 minutes. Thereafter, the membrane was immersed in the sucrose solution and shaken slowly for 5 minutes. The excess moisture on the membrane was then absorbed and dried at room temperature to make the immunochromatographic migration membrane (30). Using this membrane, the immunochromatographic strip (A) was prepared as shown in Figs. 3 and 4.

### (Assay Procedures)

To the sample pad (10) of the immunochromatographic strip (A) prepared above, the canine NT-proANP (0.5 µg/0.5 ml of the sample migration buffer) was poured, developing the canine NT-proANP to the conjugate pad (20) with the colloidal gold-labeled anti-canine NT-proANP (68-98) antibody (clone #3D2) coated thereon. The sample migration buffer migrated from the sample pad (10) to the conjugate pad (20) enabled conjugating the canine NT-proANP with the colloidal gold-labeled anti-canine NT-proANP (68-98) antibody by the antigen-antibody reaction. The resulting sample migration buffer flowed continuously to the migration membrane (30) and then continued to flow and develop to the test line (32) of the migration membrane (30) which was in turn coated with the anti-canine NT-proANP (31-67) antibody (clone #2E3). In the test line (32), the canine NT-proANP-colloidal gold-anti-canine NT-proANP antibody conjugate in the sample migration buffer was then caused to further conjugate with the anti-canine NT-proANP (31-67) antibody (clone #2E3) coated in the test line (32) by the antigen-antibody reaction, resulting in the formation of a band on the test line.

### (Assay Results)

As shown in Fig. 5, the result of the above quick test confirmed the band of the canine NT-proANP in the test line of the immunochromatographic strip, thereby indicating the presence of the canine NT-proANP in the sample as the antigen of question

### INDUSTRIAL APPLICABILITY

The anti-canine NT-proANP antibodies and the immunological measurement method are useful for a method for the detection of heart diseases and infections of companion mammals such as dogs and cats, including, for example, mitral valve insufficiency and heart failure as well as filariasis.

## Claims

1. An anti-canine N-terminal pro-atrial natriuretic peptide antibody recognizing a particular site of an amino acid sequence of canine NT-proANP different from the corresponding amino acid sequence of human NT-proANP.

2. The anti-canine NT-proANP antibody as claimed in claim 1, comprising an anti-canine NT-proANP antibody recognizing a partial portion or a whole portion of amino acid sequence 31 to 67 of canine NT-proANP and/or an anti-canine NT-proANP antibody recognizing a partial portion or a whole portion of amino acid sequence 68 to 98 thereof.

3. The anti-canine NT-proANP antibody as claimed in claim 1, wherein the anti-canine NT-proANP (31-67) antibody is an anti-canine NT-proANP antibody recognizing amino acid sequence 32 to 40 (SQ ID NO:2) of canine NT-proANP and the anti-canine NT-proANP (68-98) antibody is an anti-canine NT-proANP antibody recognizing amino acid sequence 74 to 82 (SQ ID NO:3) thereof.

4. The anti-canine NT-proANP antibody as claimed in claim 3, comprising the anti-canine NT-proANP (32-40) antibody produced from hybridoma strain having clone number (2E3) and deposited as NITE P-1318 and the anti-canine NT-proANP (74-82) antibody produced from hybridoma strain having clone number (3D2) and deposited as NITE P-1319.

5. The anti-canine NT-proANP antibody as claimed in claim 1, wherein the anti-canine NT-proANP antibody is a polyclonal antibody or a monoclonal antibody.

6. The anti-canine NT-proANP antibody as claimed in claim 5, wherein the anti-canine NT-proANP antibody is a monoclonal antibody.

7. An immunological measurement method for immunologically measuring canine N-terminal pro-atrial natriuretic peptide using an anti-canine N-terminal pro-atrial natriuretic peptide antibody recognizing a particular site of an amino acid sequence of canine NT-proANP different from the corresponding amino acid sequence of human NT-proANP.

8. The immunological measurement method as claimed in claim 7, wherein the anti-canine NT-proANP antibody is an anti-canine NT-proANP (31-67) antibody recognizing a partial portion or a whole portion of amino acid sequence 31 to 67 of canine NT-proANP and/or an anti-canine NT-proANP (68-98) antibody recognizing a partial portion or a whole portion of amino acid sequence 68 to 98 thereof.

9. The immunological measurement method as claimed in claim 8, wherein the anti-canine NT-proANP (32-40) antibody is produced from hybridoma strain having clone number (2E3) and deposited as NITE P-1318 and the anti-canine NT-proANP (74-82) antibody is produced from hybridoma strain having clone number (3D2) and deposited as NITE P-1319.

10. The immunological measurement method as claimed in claim 7, wherein the anti-canine N-terminal pro-atrial natriuretic peptide antibody is a polyclonal antibody or a monoclonal antibody.

11. The immunological measurement method as claimed in claim 7, wherein the canine NT-proANP is measured in a sample of body fluid, blood, serum or plasma of a non-human mammal.

12. The immunological measurement method as claimed in claim 7, wherein the canine NT-proANP is measured in a sample of body fluid, blood, serum or plasma, containing aprotinin or EDTA.

13. A method for detecting a heart disease or an infection of a non-human mammal using the anti-canine NT-proANP antibody as claimed in claim 1 and the immunological measurement method as claimed in claim 7.

14. The method as claimed in claim 13, wherein the heart disease of the non-human mammal is mitral valve insufficiency or heart failure and the infection thereof is filariasis.

15. An immunologically measuring kit for immunologically measuring canine N-terminal pro-atrial natriuretic peptide (NT-proANP) as an antigen in a sample of a non-human mammal such as a dog, comprising a support member, a first antibody to be immobilized on the support member, an enzyme-labeled second antibody or a mix of a biotin-labeled second antibody and an enzyme-labeled avidin to be conjugated with the antigen immobilized on the support member, and a chromogenic substrate converting the enzyme of the enzyme-labeled second antibody to generate a luminescence therefrom and measuring an absorbance of the luminescence,
wherein the first antibody is different from the second antibody and selected from anti-canine NT-proANP recognizing the amino acid sequence 31 to 67 of canine NT-proANP and anti-canine NT-proANP recognizing the amino acid sequence 68 to 98 thereof,

16. The immunologically measuring kit as claimed in claim 15, wherein the enzyme of the enzyme-labeled second antibody of the enzyme-labeled avidin is a peroxidase, preferably horseradish peroxidase, and the avidin of the enzyme-labeled avidin is avidin or streptoavidin.

17. An immunological measurement method using the immunological measuring kit as claimed in claim 16, comprising:
adding a sample of a non-human mammal such as a dog containing an antigen of question to the supporting member such as a plate, preferably wells of a microplate, on which a first antibody is immobilized, the first antibody being selected from anti-canine NT-proANP (31-67) antibody recognizing amino acid sequence 31 to 67 of canine NT-proANP and anti-canine NT-proANP (68-98) antibody recognizing amino acid sequence 68 to 98 thereof,
adding an enzyme-labeled second antibody or a mix of a biotin-labeled second antibody with an enzyme-labeled avidin to the antigen immobilized on the support member, conjugating the antigen-conjugated first antibody with the second antibody, resulting in the formation of a first antibody-antigen-second antibody conjugate, the second antibody being different from the first antibody, selected from the anti-canine NT-proANP (31-67) antibody, preferably anti-canine NT-proANP (32-40) antibody, and anti-canine NT-proANP (68-98) antibody, preferably anti-canine NT-proANP (74-82) antibody,
adding the chromogenic substrate against the enzyme conjugated with the second antibody to the conjugate, and
measuring an absorbance of the enzyme converted by the chromogenic substrate.

18. An immunologically measuring kit using an immunochromatographic means for immunochromatographically measuring canine NT-proANP as an antigen in a sample of a non-human mammal such as a dog, the immunochromatographic means comprising:
an immunochromatographic strip comprising a sample pad, a conjugate pad, and a migration membrane with a test line provided in this order in the direction of a lateral flow of the sample, wherein:
the sample pad for receiving the sample;
the conjugate pad containing a first antibody labeled on a coloring substance, such as colored particles, fluorescent or magnetic particles, preferably colloidal colored metal particles, more preferably colloidal gold particles, and
the test line of the migration membrane with a second antibody immobilized thereon, the second antibody being different from the first antibody and selected from the anti-canine NT-proANP (31-67) antibody, preferably anti-canine NT-proANP (32-40) antibody, and anti-canine NT-proANP (68-98) antibody, preferably anti-canine NT-proANP (74-82) antibody,
wherein the antigen in the sample added to the sample pad is conjugated with the first antibody labeled with the coloring substance on the conjugate pad, resulting in the formation of a coloring substance-labeled first antibody-antigen conjugate by antigen-antibody reaction, and
wherein the coloring substance-labeled first antibody-antigen conjugate is further conjugated with the second antibody immobilized on the test line of the migration membrane, resulting in the formation of a first antibody-antigen-second antibody conjugate which changes color which is then visualized.

19. An immunological measurement method for immunologically measuring canine NT-proANP in a sample of a non-human mammal as an antigen, using the immunologically measuring kit as claimed in claim 19, the immunochromatographic means comprising an immunochromatographic strip having a sample pad, a conjugate pad, and a migration membrane provided with a test line, wherein:
the sample containing the antigen is added to the sample pad of the immunochromatographic strip,
the antigen contained in the sample is conjugated with the first antibody labeled with the coloring substance on the conjugate pad, forming a coloring substance-labeled antibody-antigen conjugate, and
the antigen of the coloring substance-labeled first antibody-antigen conjugate contained in the sample is further conjugated with the second antibody immobilized on the test line of the migration membrane, forming a conjugate of the second antibody with the antigen of the coloring substance-immobilized first antibody-antigen conjugate, and resulting in the test line of the migration membrane changing color and visualizing the presence of the antigen as the object of measurement in the sample,
wherein the first antibody is different from the second antibody and each of the first and second antibodies is selected from the anti-canine NT-proANP (31-67) antibody, preferably anti-canine NT-proANP (32-40) antibody, and anti-canine NT-proANP (68-98) antibody, preferably anti-canine NT-proANP (74-82) antibody.


SEQUENCE LISTING

<110> Shibayagi Co., Ltd.

<120> Anti-canine N-terminal atrial natriuretic peptide antibody, and
immunological measurement method and immunological measurement kit using the
same

<160> 3

<170> PatentIn version 3.5.1

<210> 1
<211> 98
<212> PRT
<213> Canis familiaris

<400> 1

Asn Pro Val Tyr Gly Ser Val Ser Asn Ala Asp Leu Leu Asp Phe
1               5                   10                  15

Lys Asn Leu Leu Asp Arg Leu Glu Asp Lys Met Pro Leu Glu Asp
                20                  25                  30

Glu Ala Glu Ser Pro Gln Ala Leu Ser Glu Gln Asn Ala Glu Ala
                35                  40                  45

Gly Ala Ala Leu Ser Pro Leu Pro Glu Val Pro Pro Trp Thr Gly
                50                  55                  60

Glu Val Ser Pro Ala Gln Arg Asp Gly Gly Ala Leu Gly Arg Ser
                65                  70                  75

Pro Trp Asp Ser Ser Asp Arg Ser Ala Leu Leu Lys Ser Lys Leu
                80                  85                  90

Arg Ala Leu Leu Ala Ala Pro Arg
                95          98

<210> 2
<211> 9
<212> PRT
<213> Canis familiaris

<400> 2

Ala Glu Ser Pro Gln Ala Leu Ser Glu
1               5               9

<210> 3
<211> 9
<212> PRT
<213> Canis familiaris

<400> 3

Arg Ser Pro Trp Asp Ser Ser Asp Arg
1               5               9